# FASCICULE DE BREVET EUROPEEN

(11) **EP 1 868 536 B1**
(45) Date de publication et mention de la délivrance du brevet: **17.11.2010**
(21) Numéro de dépôt: 06743805.1
(22) Date de dépôt: 12.04.2006
(51) Int. Cl.: A61F 2/42

(54) **DISPOSITIF D'OSTEOSYNTHESE INTRAMEDULLAIRE DE DEUX PARTIES D'OS, NOTAMMENT DE LA MAIN ET/OU DU PIED**
INTRAMEDULLÄRE OSTEOSYNTHETISCHE VORRICHTUNG VON ZWEI KNOCHENTEILEN, INSBESONDERE HAND UND/ODER FUSS
INTRAMEDULLAR OSTEOSYNTHETIC DEVICE OF TWO BONE PARTS, IN PARTICULAR OF THE HAND AND/OR FOOT

(30) Priorité: 14.04.2005 FR 0550957
(43) Date de publication de la demande: 26.12.2007
(60) Demande divisionnaire: 10003892.6
(73) Titulaire: Memometal Technologies, 35170 Bruz (FR); Augoyard, Marc, 69160 Tassin la Demi Lune (FR); Peyrot, Jacques, 69160 Tassin la Demi Lune (FR); Meusnier, Tristan, 42000 St Etienne (FR)
(72) Inventeur: PRANDI, Bernard, F-35700 Rennes (FR)
(74) Mandataire: Benech, Frédéric
(86) Numéro de dépôt international: PCT/FR2006/050345
(87) Numéro de publication internationale: WO 2006/109004

(56) Documents cités:
- EP-A- 1 300 122
- FR-A- 2 783 702
- FR-A- 2 787 313
- FR-A- 2 794 019
- GB-A- 2 119 655
- US-A- 3 466 669
- US-A- 3 681 786
- US-A- 3 824 631
- US-A- 4 364 382
- US-A- 5 425 777

## Description

L'invention se rattache au secteur technique des implants orthopédiques, notamment pour arthrodèses et ostéosynthèses.

On rappelle que le but d'une arthrodèse est d'obtenir une très bonne stabilité aussi bien primaire que secondaire et de mettre, ou de maintenir, en compression deux parties d'os ou fragments osseux qu'il convient d'immobiliser. La stabilité est critique pour obtenir la consolidation, tout en minimisant les problèmes annexes tels que douleur, gonflement... L'effet de compression permet de consolider plus rapidement l'ostéotomie dans la position choisie par le chirurgien lors de l'opération.

Différentes solutions techniques ont été proposées pour réaliser une arthrodèse, notamment au niveau du pied, de la main, du poignet, ...On peut citer, par exemple, les agrafes basiques sans mémoire de forme qui n'assurent pas une compression, à l'inverse des agrafes à mémoire qui permettent de mettre en compression les deux parties d'os à consolider, ce qui correspond au but recherché.

Toutefois, pour obtenir une stabilité satisfaisante, il est nécessaire de mettre deux, voire trois agrafes, dans des plans différents. Il en résulte un encombrement important, ce qui limite les applications (articulation métacarpo-phalangienne par exemple).

On a proposé également des plaques et vis extramédullaires qui nécessitent un encombrement relativement important. Il est à cet égard difficilement envisageable de les miniaturiser, ce qui pourrait générer des problèmes de tenue et de rigidité.

**Cet état de la technique peut être illustré par l'enseignement du brevet** US 4,364,382 **qui décrit une plaque de section méplate dont les bords longitudinaux présentent une série de dentures.**

Certains types de vis peuvent être utilisées en intramédullaire, mais génèrent des difficultés de positionnement (passage par la pulpe notamment).

On peut également utiliser des broches qui présentent un encombrement réduit. Toutefois, la stabilité obtenue n'est pas satisfaisante et il est nécessaire de procéder à un retrait. **Une solution de ce type ressort de l'enseignement du brevet** GB 2119655**.**

**Le brevet** FR 2.794.019 **décrit un implant d'ostéosynthèse destiné au traitement de tout type d'os, en particulier des os longs et de petits os, constitué par un corps de forme générale de révolution comprenant une partie constituée par un enroulement hélicoïdal d'un fil en matériau déformable, pour le positionnement d'un moyen d'appui en regard des fragments osseux.**

**Le brevet** FR 2.787.313 **concerne également un implant d'ostéosynthèse constitué par un corps de forme générale de révolution pour permettre l'introduction de l'implant dans le fragment osseux par contraction et son appui contre le fragment dans un état d'expansion**

Le document US 3,824,631 décrit un implant d'ostéosynthèse qui sert de pont dans un joint entre deux os et formé de métal ayant une forme creuse et muni à une extrémité d'une bifurcation pour pouvoir recevoir le canal médullaire de deux métacarpes. A l'extrémité opposée, une extension est prévue pour implantation dans le radius.

Le document EP 1 300 122 A2 décrit un implant ayant deux bras allongés reliés entre eux par un membre permettant la flexibilité entre les deux bras. Le membre reliant ayant deux zones concaves chacune située d'un côté opposé du membre.

Enfin, on connaît des clous intramédullaires mais ces derniers nécessitent un agrafage complémentaire afin de les bloquer en rotation.

L'invention s'est fixée pour but de remédier à ces inconvénients d'une manière simple, sûre, efficace et rationnelle.

Le problème que se propose de résoudre l'invention est de permettre la fixation de deux parties d'os l'une à l'autre, d'une manière rigide avec une compression dynamique et rétentive afin d'obtenir une ostéosynthèse fiable et rapide.

Pour résoudre un tel problème, il a été conçu et mis au point un élément d'arthrodèse intramédullaire **conforme aux caractéristiques définies dans la revendication 1.**

L'invention trouve une application particulièrement avantageuse, pour la réalisation d'arthrodèses au niveau des phalanges proximales et moyennes, pour les articulations interphalangiennes proximales et les articulations interphalangiennes distales, au niveau de la main et/ou au niveau du pied.

L'invention est exposée ci-après plus en détail à l'aide des figures des dessins annexés dans lesquels :
- la figure 1 est une vue en plan à caractère schématique montrant la mise en place de l'élément d'arthrodèse intramédullaire selon l'invention entre une phalange proximale et une phalange moyenne pour assurer le blocage de l'articulation interphalangienne proximale ;
- la figure 2 est une vue en plan d'un exemple de réalisation de l'élément d'arthrodèse au moment de son introduction ;
- la figure 3 est une vue correspondant à la figure 2 montrant l'élément d'arthrodèse après son implantation pour réaliser la compression ;

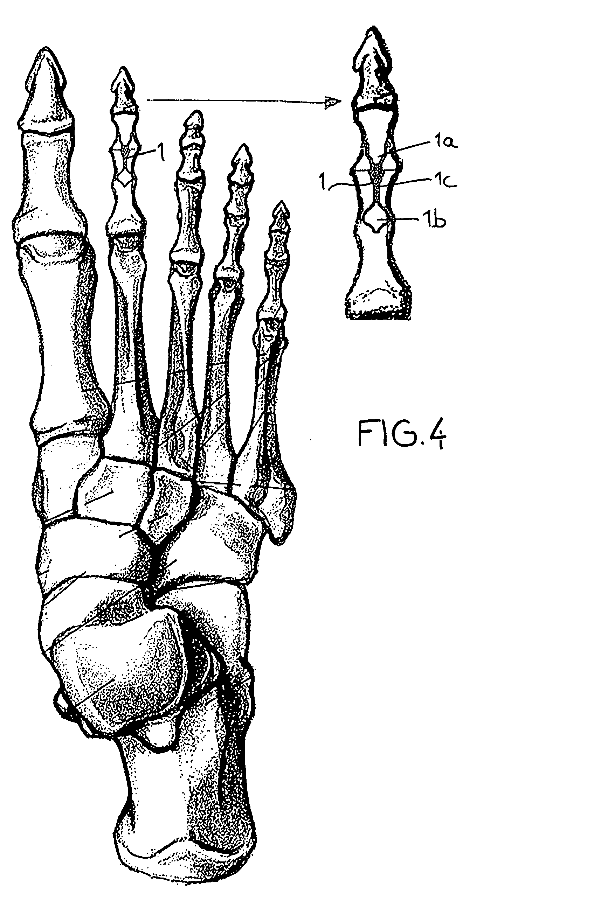

- la figure 4 montre la mise en place de l'élément selon l'invention au niveau d'un orteil

L'élément d'arthrodèse selon l'invention est constitué par un corps de forme allongée désigné dans son ensemble par (1). Chacune des extrémités du corps est conformée pour réaliser une zone d'ancrage (1a) à une zone d'ancrage (1b).

Entre les deux zones d'ancrage (1a) et (1b), est formée au moins une zone médiane (1c) apte à résister aux contraintes de cisaillement et de flexion. Généralement, les contraintes de cisaillement et de flexion s'exercent au niveau du foyer osseux à consolider. La forme de cette zone médiane (1c) est adaptée à la forme interne de l'os. Sa longueur est déterminée afin d'autoriser un léger décalage dans le centrage.

A titre indicatif nullement limitatif, cette zone médiane peut avoir une section rectangulaire de 2 à 3 mm x 1 à 1,5 mm environ et une longueur de 3 à 5 mm environ (pour le pied et la main).

Les zones d'ancrage (1a) et (1b) sont conformées pour éviter tout mouvement de rotation, résister à une sollicitation en traction, et maintenir la compression manuelle mise au moment de la pose par le chirurgien afin de réduire le foyer. Pour obtenir ce résultat, les zones d'ancrage (1a) et (1b) sont réalisées dans un matériau à mémoire de forme pour être déformées par effet thermique (mémoire tiède) ou par effet mécanique (superélasticité). Le but recherché, au niveau des zones d'ancrage, en considérant, d'une part, leur profil et, d'autre part, la nature du matériau les constituant est de permettre une introduction dans les parties d'os, notamment par voie dorsale sans abord pulpaire, d'une part, puis d'assurer un ancrage dans ladite partie d'os afin d'obtenir ou de maintenir l'effort de compression recherché, d'autre part. Les zones d'ancrage (1a) et (1b) sont identiques ou non, selon le type d'os et sa morphologie.

En fonction du type d'arthrodèse réalisée, c'est-à-dire de la nature des articulations interphalangiennes à bloquer par exemple, les zones d'ancrage (1a) et (1b) peuvent présenter différentes formes de réalisation.

Par exemple, l'une des zones d'ancrage (1a) présente deux pattes ou ailettes aptes à être écartées sous un effet thermique par exemple. Ou bien, ces zones d'ancrage (1a) peuvent présenter une seule patte ou tige apte à être recourbée sous l'effet de la mémoire du matériau la constituant. Ou bien, la zone d'ancrage (1b) présente, dans son épaisseur, une lumière pour permettre une déformation par élasticité, sous un effet thermique par exemple, et maintenir la position par une pression sur la longueur de l'os.

Suivant une autre caractéristique de l'invention, pour tenir compte de l'anatomie des différentes phalanges par exemple, à savoir le rétrécissement interne de l'os (forme sablier), la zone médiane (1c) est reliée à au moins l'une des zones d'ancrage (1b) par une zone de liaison (1d) plus fine.

On renvoie aux figures des dessins qui montrent un exemple de réalisation d'un élément d'arthrodèse intramédullaire.

Dans cet exemple de réalisation, le corps (1) présente, à l'une de ses extrémités, une zone d'ancrage (1a) sous forme de deux pattes ou ailettes (1a1) - (1a2). Cette zone d'ancrage (1a) est prolongée par la zone médiane (1c) de forme générale, vue en plan, sensiblement triangulaire. La zone médiane (1c) est raccordée à l'autre zone d'ancrage d'extrémité (1b) par une zone de liaison (1d) de forme générale, vue en plan, sensiblement rectangulaire. La zone d'ancrage (1b) présente, dans son épaisseur, une lumière de forme générale sensiblement oblongue (1b1).

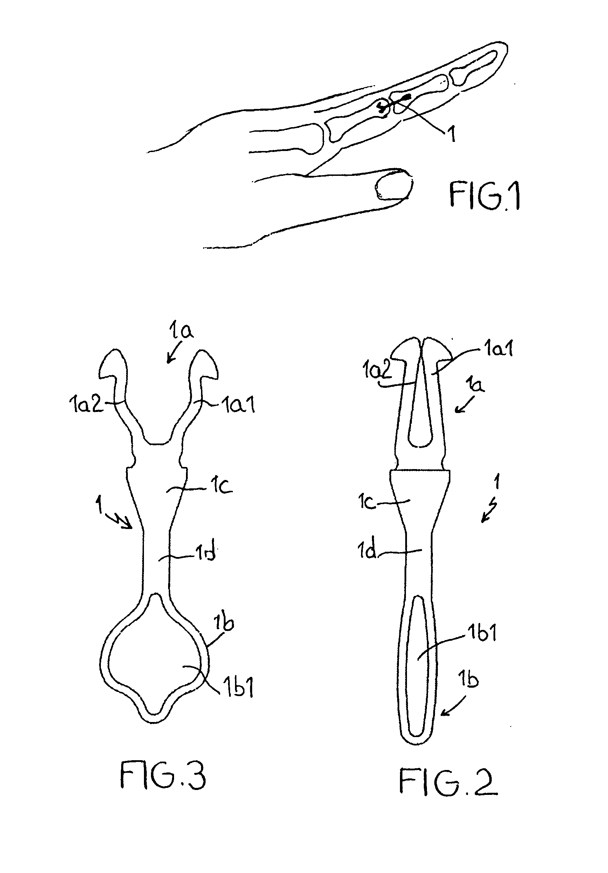

On renvoie à la figure 2 qui montre l'élément au moment de son introduction, c'est-à-dire avant écartement des pattes (1a1) - (1a2), et ouverture de la lumière (1b1). Par exemple, cette configuration est obtenue lorsque l'ensemble de l'élément est soumis à une basse température très inférieure à celle du corps humain par exemple. Inversement, après implantation (figure 3), sous l'effet de la chaleur du corps, les pattes (1a1) et (1a2) sont écartées, de même que la lumière (1b1) ce qui provoque, d'une manière concomitante, une déformation de la zone d'ancrage (1b).

A noter que le profil de la zone médiane (1c) évite l'enfoncement quand on vient refermer le foyer.

Dans une forme de réalisation en variante, la zone de liaison (1d) peut être fendue pour bénéficier d'un effet de gonflement par mémoire de forme et de renforcement de l'ancrage dans la zone diaphysaire.

On rappelle que l'élément selon l'invention est particulièrement bien adapté pour le traitement de la pathologie « orteil en griffe ou en marteau » en réalisant une arthrodèse au niveau des phalanges P1 et P2 sur les rayons 2 à 5, en observant que de telles applications ne doivent pas être considérées comme limitatives, moyennant des adaptations essentiellement dimensionnelles (réimplantations de doigt, arthrodèse de l'articulation interphalangienne distale et de l'articulation interphalangienne proximale de la main, arthrodèse MP du gros orteil).

Bien évidemment, l'ensemble de l'élément d'arthrodèse selon l'invention peut présenter des caractéristiques de construction aptes à améliorer notamment l'ancrage et la compression.

Par exemple :
- des crans sur les pattes d'un des côtés pour un meilleur ancrage dans l'os spongieux ;
- des pattes ondulées implantées (droites avant implantation) pour permettre un raccourcissement et donc une mise en compression du foyer d'arthrodèse supplémentaire par rapport à un simple maintien ;
- une zone centrale conique pour éviter un enfoncement non désiré de l'implant au moment où l'on vient fermer le foyer.

A titre indicatif, la mémoire utilisée est préférentiellement de la mémoire tiède, de sorte qu'un chauffage n'est pas nécessaire car il n'y a pas d'accès. Le début d'ouverture s'effectue aux environs de 15 à 20°C, tandis que la fin s'effectue aux environs de 30 à 35°C.

La technique opératoire demeure classique.

## Revendications

1. Dispositif d'ostéosynthèse intramédullaire de deux parties d'os, notamment de la main et/ou du pied, constitué par un corps (1) de forme allongée et de section transversale méplate présentant, successivement, à partir de l'une de ses extrémités, une zone d'ancrage (1a) coopérant avec l'une des parties d'os à immobiliser, une zone médiane (1c) apte à résister aux contraintes de cisaillement et de flexion, et une zone d'ancrage (1b) dans l'autre partie d'os à immobiliser, **caractérisé en ce que** :
- chacune desdites zones d'ancrage (1a) et (1b) **est** réalisées dans un matériau **à mémoire de forme** apte à permettre leur déformation , afin de permettre une introduction dans les parties d'os sans abord pulpaire, puis assurer un ancrage dans lesdites parties d'os en évitant tout mouvement de rotation, en résistant à la traction et en maintenant un effort de compression ;
- **la zone d'ancrage (1a) coopérant avec l'une des parties d'os à immobiliser présente deux pattes ou ailettes (1a1) et (1a2) aptes à être écartées sous l'effet de la déformation, par mémoire de forme ;**
- **la zone d'ancrage (1b) dans l'autre partie d'os à immobiliser présente, dans son épaisseur, une lumière (1b1) pour permettre une déformation par élasticité sous l'effet de la mémoire de forme et un blocage par gonflement.**

2. Dispositif selon la revendication 1, **caractérisé en ce que** la zone médiane (1c) est reliée à la zone d'ancrage (1b) par une zone de liaison (1d) de section réduite.

3. Dispositif selon la revendication 1, **caractérisé en ce que** la zone médiane (1c) est **de forme générale, vue en plan, sensiblement triangulaire,** pour éviter l'enfoncement quand on vient refermer le foyer de fracture.

## Claims

1. Intramedullary osteosynthesis device for two portions of bone, particularly of the hand and/or the foot, constituted by a body (1) of elongated form and with a flat cross section having, in succession, starting from one of its ends, an anchorage zone (1a) cooperating with one of the portions of bone to be immobilised, a median zone (1c) capable of withstanding shear stress and bending stress, and a zone (1b) for anchorage in the other portion of the bone to be immobilised, **characterised in that**:
- each of said anchorage zones (1a) and (1b) is made of a shape-memory material capable of permitting their deformation, so as to allow insertion into the portions of bone without an approach via the pulp, then to provide anchorage in said portions of bone while preventing any rotary movement, while resisting traction and while maintaining a compressive stress;
- the anchorage zone (1a) co-operating with one of the portions of bone to be immobilised has two tabs or fins (1a1) and (1a2) capable of being moved aside under the effect of deformation, by shape memory;
- the anchorage zone (1b) in the other portion of bone to be immobilised has, within its thickness, a slot (1b1) to allow deformation by elasticity under the effect of shape memory, and locking by bulging.

2. Device according to claim 1, **characterised in that** the median zone (1c) is linked to the anchorage zone (1b) by a connecting zone (1d) of reduced section.

3. Device according to claim 1, **characterised in that** the median zone (1c) is of generally substantially triangular shape, in plan view, to avoid penetration when the fracture site is reclosed.

## Patentansprüche

1. Intramedulläre osteosynthetische Vorrichtung von zwei Knochenteilen, Insbesondere der Hand und/oder des Fußes,
bestehend aus einem Körper (1) von länglicher Form und einem flachen Querschnitt mit aufeinanderfolgend, beginnend an einem seiner Enden, einer Verankerungszone (1a), welche mit einem der Knochenteile zur Immobilisierung zusammenwirkt, einer medianen Zone (1c), die ausgebildet ist, Scher- und Biegebeanspruchungen zu widerstehen, und einer Verankerungszone (1b) bei dem anderen Knochenteil zur Immobilisierung, **dadurch gekennzeichnet,**
**dass**:
- Jede der vorbenannten Verankerungszonen (1a) und (1b) aus einem Werkstoff mit einem Formgedächtnis besteht und so aufgebaut ist, dass sie ihre Verformung ermöglicht, damit eine Einführung in die Knochenteile ohne Berührung des Knochenmarks ermöglicht ist, gefolgt von einer Verankerung in den vorbenannten Knochenteilen, wobei eine Drehbewegung vermieden wird, einer Zugspannung widerstanden wird und eine Kompressionskraft aufrecht erhalten bleibt;
- die Verankerungszone (1a) mit einem der Knochenteile zur Immobilisierung zusammenwirkt und zwei Schenkel oder Flügel (1a1) und (1a2) aufweist, die so aufgebaut sind, dass sie mittels des Formgedächtnisses den Effekt der Deformierung ausüben;
- die Verankerungszone (1b) in dem anderen Knochenteil zur Immobilisierung über ihre Höhe einen Schlitz (1b1) aufweist, um eine Deformierung mittels Elastizität unter der Wirkung des Formgedächtnisses und eine Arretierung durch eine Aufweitung zu erlauben.

2. Vorrichtung nach Anspruch 1,
**dadurch gekennzeichnet,**
**dass** die mediane Zone (1c) mit der Verankerungszone (1b) über eine Verbindungszone (1d) mit reduziertem Querschnitt verbunden ist.

3. Vorrichtung nach Anspruch 1,
**dadurch gekennzeichnet,**
**dass** die mediane Zone (1c) von einer im Wesentlichen in Draufsicht gesehen dreieckigen Form ist, um ein Eindringen zu vermeiden, wenn die Bruchstelle wieder verschlossen wird.
